# EUROPEAN PATENT APPLICATION

(11) **EP 1 596 196 A2**
(43) Date of publication of application: **16.11.2005**
(21) Application number: 05010266.4
(22) Date of filing: 11.05.2005
(51) Int. Cl.: G01N 33/00

(54) **Protein interaction chip**

(30) Priority: 12.05.2004 JP 2004142275
(71) Applicant: Hitachi High-Technologies Corporation, Tokyo 105-8717 (JP)
(72) Inventor: Kanda, Katsuhiro, Hitachinaka-shi, Ibaraki 312-8504 (JP); Sasakura, Yukie, Hitachinaka-shi, Ibaraki 312-8504 (JP)
(74) Representative: Strehl Schübel-Hopf & Partner

(57) **Abstract**

It is an object of the present invention to conduct function analysis of proteins arranged with high density on a solid phase in a high-throughput manner, regardless of protein varieties. The present invention is characterized in that it immobilizes target proteins on a substrate while maintaining the structures and the functions thereof, and that it carries out interaction analysis of such proteins. For example, target proteins are prepared in a state where one end of each protein is fused to a peptide as a tag or to a polypeptide by genetic engineering methods. Then, the target proteins are bonded to the substrate via a layer formed thereon, comprising anti-tag antibodies. The target proteins are arrayed while they are maintained in a liberated state on a solid phase, and the interaction analysis is carried out. According to the present invention, the interaction analysis of target proteins can be carried out without affecting the unstable structures and the functions thereof. For example, the present invention can be applied as a platform for interaction analysis, screening, quantitation, expression profiling, or the like, regarding proteins.

## Description

### BACKGROUND OF THE INVENTION

### Field of the Invention

The present invention relates to an anti-tag antibody chip for collectively conducting function analysis of proteins arranged with high density on a solid phase in a high-throughput manner and to a related technique thereof.

### Background Art

As genomics has progressed regarding various species, vast quantities of nucleic acid sequences have been accumulated on a worldwide scale. Inside a living thing, on the basis of the nucleic acid sequences, proteins are synthesized through the processes of transcription and translation, and various activities of the living thing are maintained by the functions thereof. In recent years, with the enrichment of genomic information, structure and function analyses of proteins, which have a primary role among the functional molecules in a living body, have been actively conducted as proteomics or proteome analysis.

Examples of protein function analysis conducted in a liquid phase include an enzyme and an inhibitor assay experiment. The former is an experiment in which an enzyme and a substrate in a test tube are caused to react in a solution in order to examine the optimum conditions upon measuring enzyme activity. The latter is an experiment in which a substance is added to a reaction liquid thereof so as to search for a substance that inhibits an enzyme reaction.

As one example, tyrosinase (EC 1.14.18.1) engaged in melanin synthesis catalyzes a reaction of monohydroxyphenol (tyrosine, for example) → dihydroxyphenol (DOPA, for example) → quinone (DOPA quinone, for example). Thus, by causing a tyrosinase solution and a tyrosine solution to react, the synthesis of DOPA and DOPA quinone can be spectroscopically measured with absorbances of 280 nm and 475 nm, respectively, over the course of time. An assay protocol is established by optimizing reaction conditions, such as temperature, component, time, and pH. By adding a substance to a reaction solution, a substance that inhibits the reaction is searched for. For example, by adding kojic acid, which is an inhibitor of tyrosinase activity, results in which the synthesis of DOPA and DOPA quinone is restrained can be achieved.

In contrast to the aforementioned protein function analysis conducted in a liquid phase, examples of the proteomics on a solid phase include a structure analysis technique for proteins via a mass spectrometry method, for example. A technique of immobilizing a protein onto a substrate has been proposed. However, it is characterized in that the functions of proteins need not be maintained for structure analysis to take place. Also, protein-protein interaction analysis via surface plasmon resonance and quartz crystal microbalance methods has been attempted. However, none of the methods have been capable of interaction analysis concerning a certain number of samples in a collective manner.

In particular, in order to conduct function analysis of proteins arranged with high density on a solid phase in a high-throughput manner, a device for immobilization onto a substrate while maintaining unstable protein structures and functions is necessary. Examples of such immobilization of proteins onto a substrate include methods of physisorption, bonding via the functional groups of various compounds, embedding in a polymer layer, and the like. Also, such examples include a method of capturing proteins using host/guest molecules, such as calixarene. Any of such methods are capable of immobilizing proteins onto a substrate. However, the maintenance of the protein structures and functions are not considered.

At present, there are some products referred to as "protein chips" on the market. For example, HydroGel (PerkinElmer, Inc.) and Power Matrix Slide (Full Moon BioSystems, Inc.) are devices in which polymer gel is layered on a glass substrate, thereby capturing protein molecules three-dimensionally. In contrast, FAST Slide (Schleicher & Schuell, Inc.) is a product in which a nitrocellulose membrane is attached to a glass substrate. Also, chips of other companies having the surfaces of glass substrates modified with aldehyde groups, epoxy groups, amine group, or the like such that they can be used to immobilize proteins are also on the market.

As an application, a chip-based immunoassay method has been presented regarding HydroGel or FAST Slide, for example. In this method, an antibody is immobilized on the chip and the quantity of antigen that specifically bonds to the antibody is measured. However, interactions are not analyzed on such chip with measures for maintaining the structures and the functions of the antigen (target protein).

Although the protein chip is expected as one means of function analysis among various proteomics analyses, the structures and the functions of proteins cannot be maintained in many cases if the proteins are immobilized on a substrate, such as a slide glass, directly or by using a protein immobilizing reagent.

In contrast, currently, expression profiling using DNA microarrays is being actively conducted. Such expression profiling includes a comprehensive analysis of genes expressing in the living thing, followed by characterization of the living thing in accordance with the patterns thereof. Expression profiling is a technique by which the expression patterns of a patient of a certain disease are compared with those of a non-patient, for example, thereby specifying a gene relating to the disease. Also, it can be applied to the development and evaluation of medicines that normalize the gene expression of a relevant gene and inhibit the functions of translation products. However, the genes in the living thing have a primary role as information molecules, and the genes per se do not bring about disease. In the living thing, it is proteins resulting from the translation of the genes that have the role of functional molecules. Moreover, in many cases, there may not necessarily be a correlation between the expression level of genes and a given disease, so that functional changes in a living thing are expected to be evaluated more accurately by carrying out the expression profiling of the proteins than by carrying out the expression profiling of the genes.

### SUMMARY OF THE INVENTION

It is an object of the present invention to conduct function analysis of proteins arranged with high density on a solid phase in a high-throughput manner, regardless of protein varieties.

The present invention is characterized in that it immobilizes target proteins on a substrate while maintaining the structures and the functions thereof, and that it carries out interaction analysis of such proteins.

For example, target proteins are prepared in a state where one terminal of each protein is fused to a peptide as a tag or to a polypeptide by genetic engineering methods. Then, the target proteins are bonded to the substrate via a layer formed thereon, comprising anti-tag antibodies. The target proteins are arrayed while they are maintained in a liberated state on a solid phase, and the interaction analysis is carried out.

According to the present invention, the interaction analysis of target proteins can be carried out without affecting the unstable structures and the functions thereof. For example, the present invention can be applied as a platform for interaction analysis, screening, quantitation, expression profiling, or the like, regarding proteins.

### BRIEF DESCRIPTION OF THE DRAWINGS

Fig. 1 schematically shows the configuration of an anti-tag antibody chip.
Fig. 2 schematically shows a reading mechanism of an anti-tag antibody chip.
Fig. 3 schematically shows a sample dispensing mechanism of an anti-tag antibody chip.
Fig.4 shows a preparation procedure of an anti-tag antibody chip.
Fig. 5 shows a usage procedure of an anti-tag antibody chip.
Fig. 6 shows a graph of fluorescent detection results.

### DESCRIPTION OF THE PREFERRED EMBODIMENTS

In the following, the aforementioned novel features and benefits of the present invention and others of the present invention are described with reference to the drawings. However, the drawings are used mainly for description, and they do not limit the scope of the present invention.

### (Embodiment)

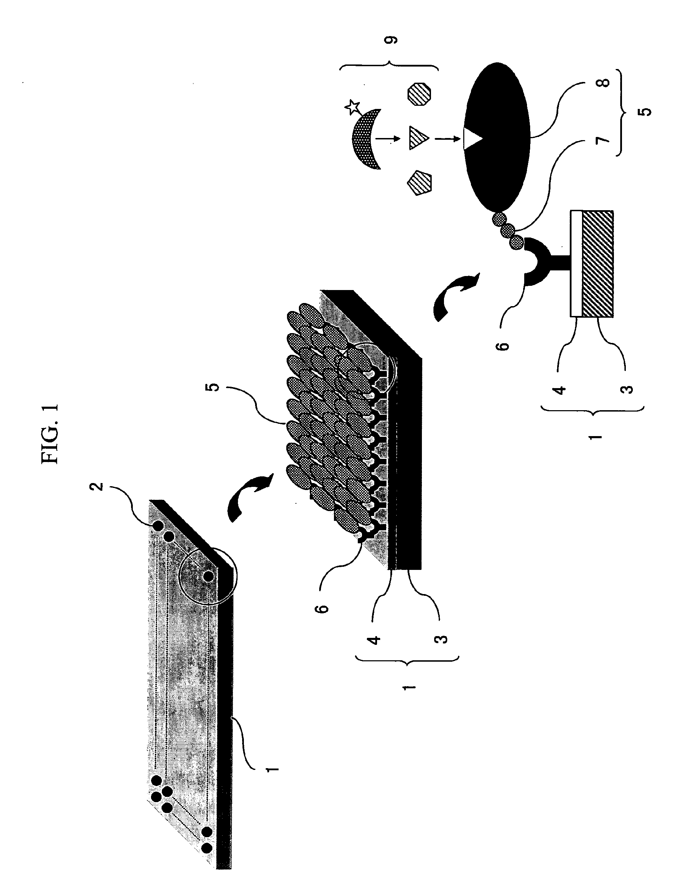

Fig. 1 schematically shows the configuration of an anti-tag antibody chip of the present embodiment, including a chip 1, an enlarged view of a portion of the chip 1, and a schematic view of a state where the chip 1 and a tag-fusion target protein 5 are bonded via an anti-tag antibody 6.

On the chip 1, m × n spots 2 are disposed lengthwise and breadthwise. The chip 1 comprises a substrate 3 and a layer of a protein immobilizing reagent 4 processed thereon. A tag-fusion target protein 5 is bonded to the layer of the protein immobilizing reagent 4 via the anti-tag antibody 6. The tag-fusion target protein 5 comprises a target protein 8 and a tag 7 and is bonded to the anti-tag antibody 6 via the tag 7 portion. The target protein 8 reacts or does not react to various types of substances, and the presence of interaction is detected through the bonding to an interaction substance 9 among various types of substances.

A protein interaction analysis apparatus of the present embodiment comprises the aforementioned anti-tag antibody chip, a dispensing mechanism for dispensing a sample including tag-fusion target proteins to the anti-tag antibody chip, and a reading mechanism for reading a labeled sample of the anti-tag antibody chip after reaction.

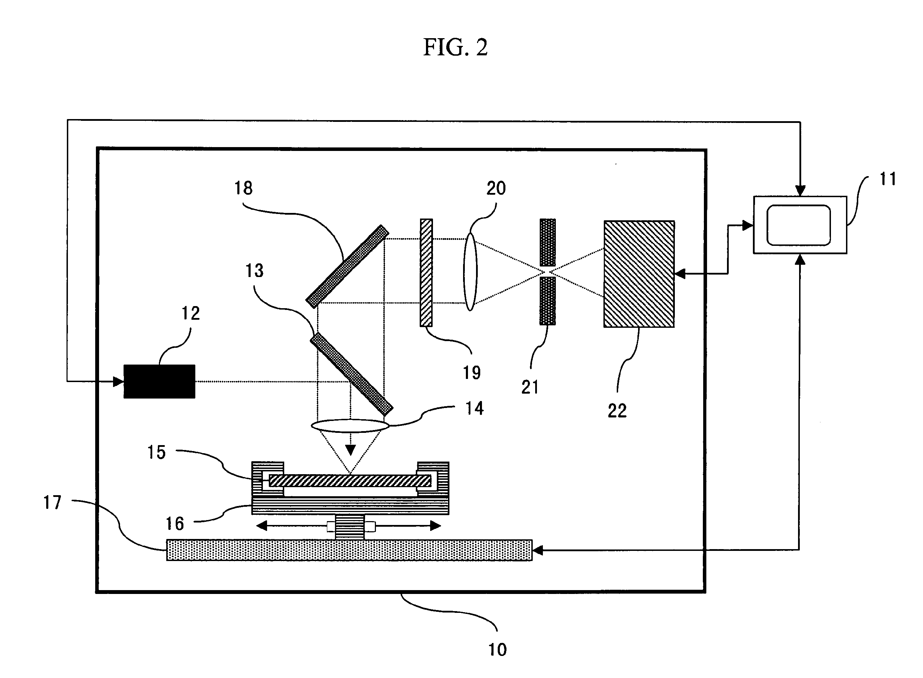

Fig. 2 schematically shows the reading mechanism of the anti-tag antibody chip. A chip 15 is held in a holder 16 and is capable of moving in the X and Y directions on a stage 17. A detection light emitted by a light source 12 is reflected via a splitter 13, condensed via a lens 14, and then the chip 15 is irradiated with such light. Reflected light from the chip 15 is passed through the lens 14 and the splitter 13, reflected via a mirror 18, passed through a filter 19, condensed via a lens 20, passed through a pinhole 21, and then detected via a detector 22. The light source 12, the stage 17, and the detector 22, for example, are controlled by a control/analysis terminal 11 outside an entire dispersing apparatus thing 10.

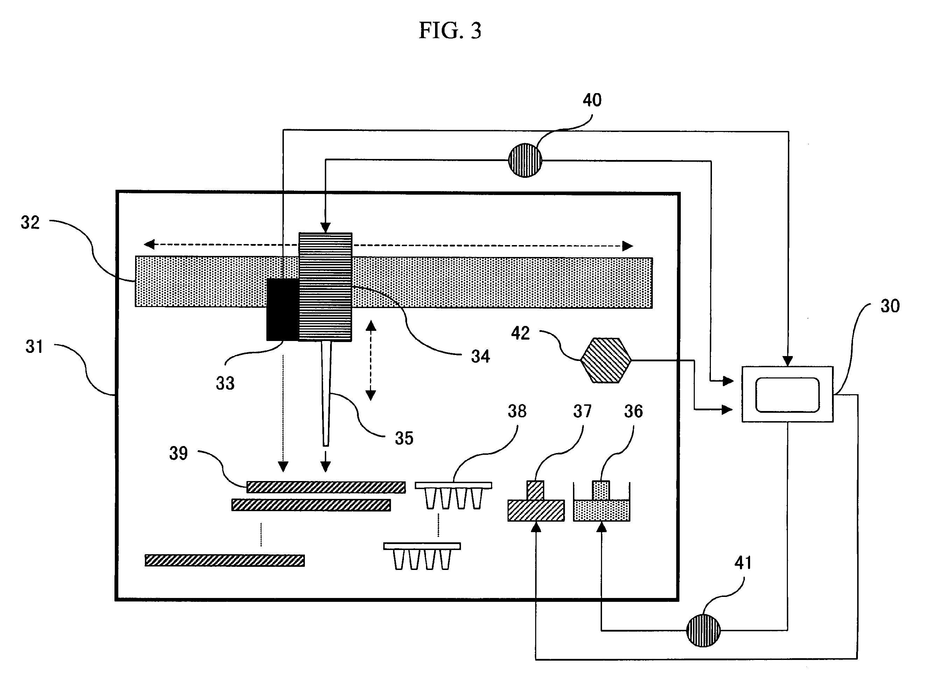

Fig. 3 schematically shows a sample dispensing mechanism of the anti-tag antibody chip. A nozzle 35 is immobilized on a head 34 that is moved on a rail 32 in accordance with a positioning apparatus 33. A sample 38 is dispensed to a chip 39 by the nozzle 35. During operation, the nozzle 35 is washed by a nozzle washing apparatus 36 and dried by a nozzle drying apparatus 37, as appropriate. A temperature/humidity sensor 42 is disposed inside an entire dispensing mechanism 31, and it is controlled by a control apparatus 30 together with the positioning apparatus 33 and pumps 40 and 41.

The target proteins are prepared in a state where peptides or polypeptides as tags are fused by a genetic engineering method. Then, the target proteins are arrayed on a solid phase via an antigen-antibody reaction between the tags and anti-tag antibodies.

In the present embodiment, differing from the case of a conventional method, a layer comprising anti-tag antibodies is formed on the chip in order to maintain the structures and the functions of the target proteins on the solid phase. Next, tag-fusion target proteins are arrayed thereon. Although the anti-tag antibodies are bonded to tags as antigens through specific recognition, they are not directly bonded to the target proteins. Therefore, the tag-fusion target proteins can be arrayed by indirect immobilization in a state where the structures and the functions thereof are not affected.

Also, since the immobilization of the tag-fusion target proteins onto the solid phase is carried out via the antigen-antibody reaction between the anti-tag antibodies forming the layer on the substrate and the tags, a binding reaction is performed by merely mixing both of them. In this case, a buffer (such as a phosphate buffer) used to dissolve protein usually is used as it is, and it is not necessary to replace it with special conditions.

Interaction analysis regarding the target proteins is conducted on the anti-tag antibody chip. The target proteins per se are maintained in a liberated state on the anti-tag antibody chip, and they are bonded to anti-tag antibodies only via the tags added to the terminal thereof, so that the target proteins are arrayed on the chip in a state where the structures and the functions thereof are maintained. Thus, according to the present embodiment, the interaction analysis of generally unstable proteins on the chip becomes possible while maintaining them in an intact state. The types of substances (such as proteins, nucleic acids, sugar, compounds, viruses, cells, or mixtures thereof) that are caused to interact with the target proteins are not limited. Also, after the substances are caused to interact with the target proteins, other substances can be additionally caused to react with the target proteins.

The types of materials of the substrate are not limited as long as they can be formed on a solid phase. Examples thereof include glass, resin, wafers, and the like, and also the types of forms are not limited; they may be a plate, a globular structure, a groove, a tubular structure, or the like. Further, the sizes thereof may be within a range that can be applied to the apparatus used.

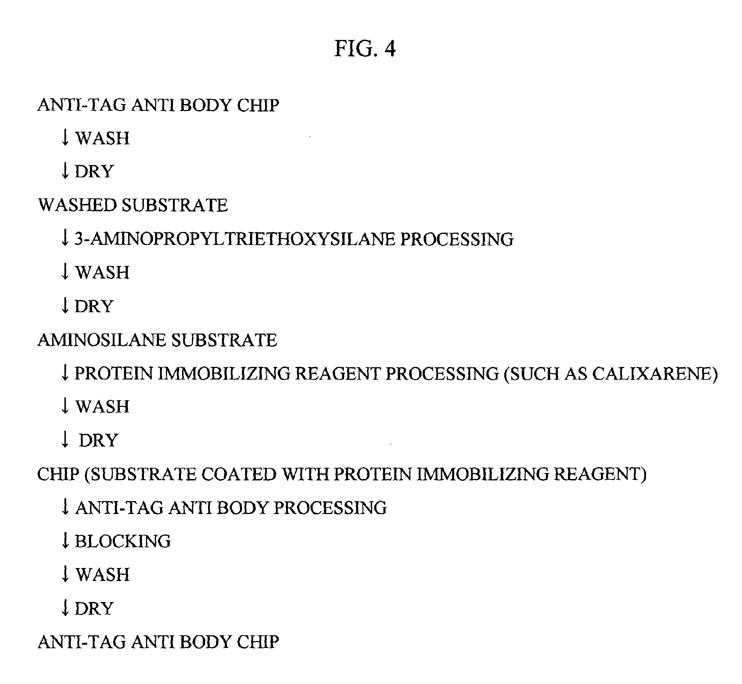

Fig.4 shows an example of a preparation procedure of the anti-tag antibody chip.

The substrate is coated with a reagent (calixarene, for example) for immobilizing proteins. On this occasion, a reagent (a silane coupling reagent such as 3-aminopropyltriethoxysilane) for mediating the bonding between a substrate surface and the protein immobilizing reagent may be used for such coating, if required. For example, in the case of calixarene, the entire chip is coated by, after the substrate is coated with 3-aminopropyltriethoxysilane, immobilizing an aldehyde group of a calixarene molecule and an amine group of a 3-aminopropyltriethoxysilane molecule via an azomethine linkage.

As the substrate for preparing anti-tag layer, a glass substrate to the surface of which a nitrocellulose membrane or polymer gel is attached can be used, or a glass substrate whose surface is modified with various types of functional groups can also be used in addition to the aforementioned substrate.

The anti-tag antibodies are immobilized on the substrate via the protein immobilizing reagent. By densely immobilizing the anti-tag antibodies so that they are in a saturated state within a spot area, a layer comprising the anti-tag antibodies is formed on the substrate. A portion where the anti-tag antibodies cannot be bonded is covered with proteins, such as bovine serum albumin (BSA) generally used for blocking, so that the target proteins do not directly contact the substrate or the protein immobilizing reagent. The tags as antigens are fused to the ends of the target proteins, so that the anti-tag antibodies recognize only the tags and are then bonded. However, the anti-tag antibodies are not bonded to the target proteins. Thus, the target proteins are arrayed on the chip by bonding to the anti-tag antibodies via the tags alone while maintaining the target proteins as they are in a liberated state.

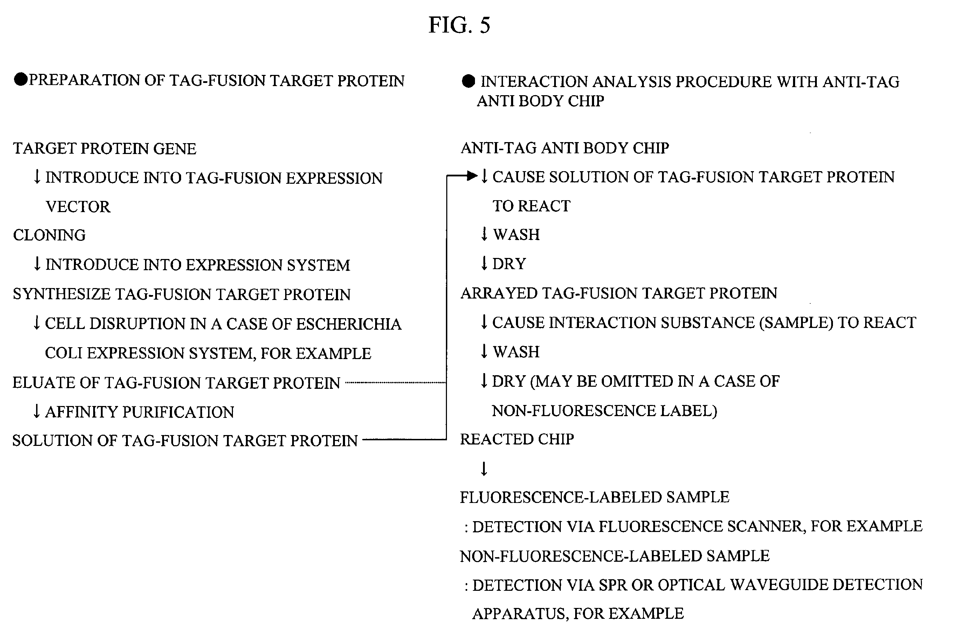

Fig. 5 shows an example of a usage procedure of the anti-tag antibody chip.

The tag-fusion target proteins are prepared by a genetic engineering method. DNA fragments for coding the target proteins are inserted in expression vectors (such as a pET vector, a pGEX vector, or the like). The expression vectors are capable of being fused to a tag at the ends of the target proteins, for example, and they are caused to overexpress in the form of the tag-fusion target proteins via an expression system suitable for the vectors. The purification of the tag-fusion target proteins is carried out by using substances (such as anti-tag antibodies or substrates) that specifically bond to the tags, and collection is performed via an affinity purification method.

In a case where it is difficult to fuse the tags to the end fields of the target proteins, the proteins may be designed so that the tags may be introduced thereinto. The present embodiment utilizes the fusion of the tags to the target proteins via genetic engineering in an arbitrary manner and provides a technique by which the target proteins are arrayed on the chip by bonding between the tags and the anti-tag antibodies in accordance with the characteristics of the target proteins.

Desirably, the types of the tags include peptides or polypeptides (proteins), since it is assumed that the tags are expressed as the tag-fusion target proteins via genetic engineering. However, in a case where proteins, such as glutathione S-transferase, are fused to the target proteins as the tags, the molecular size of the tags becomes larger, as compared with a case where peptides, such as hexa-histidine, are added as the tags. Thus, the target proteins may be subject to the influences of the tags depending on their characteristics. Therefore, the tags of the present embodiment are desirably peptides rather than polypeptides.

In recent years, genomics research has been actively carried out regarding various types of species, so that the amount of genetic information has substantially increased in comparison with available protein information. Thus, on the basis of such genetic information, the analysis of proteins resulting from translation has gradually become common. In accordance therewith, when the biosynthesis of proteins from genes is conducted using a developed genetic engineering method, in order to efficiently purify synthesized proteins, the synthesis is carried out in a state where peptides or proteins referred to as tags are fused to the ends of the proteins, and tag-fusion target proteins are affinity purified using substances (such as anti-tag antibodies, substrates, or the like) that specifically recognize the tags. The present embodiment provides a technique used for interaction analysis of synthesized tag-fusion target proteins on a solid phase without modification.

Conventionally, tag-fusion target proteins synthesized by a genetic engineering technique are, after affinity purification using affinity for tags, cleaved to the tags and target proteins using a specific protease, such as thrombin. Thereafter, an operation is conducted to remove tag fragments and undigested tag-fusion target proteins from the target protein fraction using the affinity purification method again, and so the target proteins are recovered and are used for analysis. The present embodiment does not require the dissociation of the tags and the target proteins and is characterized in that the tags are utilized as media for bonding to a chip, thereby simplifying a working process as compared with the conventional method.

As shown in Fig. 5, the present embodiment allows the affinity purification of tag-fusion target proteins expressed by a genetic engineering method simultaneously with the immobilization of the tag-fusion target proteins on a chip. In a conventional technique, the affinity purification of the expressed tag-fusion proteins, the cleavage of the tags and the target proteins using a protease, the recovery of the target proteins from a solution in which the tags and the target proteins are mixed, and the concentration of the target proteins must be conducted before interaction analysis of the target proteins. In the present embodiment, the layer comprising the anti-tag antibodies is formed on a solid phase. Thus, in a case where tag-fusion target proteins are synthesized via an expression system using *Escherichia coli,* for example, by directly distributing cell lysate to the surface of the chip, both the affinity purification of the tag-fusion target proteins and the immobilization of the tag-fusion target proteins on the chip can be simultaneously carried out without purifying the synthesized tag-fusion target proteins. Therefore, the working process can be substantially simplified and the tag-fusion target proteins can be efficiently used for interaction analysis of the target proteins on a solid phase without causing the reduction of the recovery rate of the target proteins. This can be applied as a single step method for purifying and arraying target proteins having a low expression level.

Generally, when the biosynthesis of target proteins is conducted using a genetic engineering method, the synthesis is carried out in a state where tags are fused to the ends of target proteins, and affinity purification is conducted using affinity for the tags. Thus, by unifying the types of tags, the same purification method can be applied. In this case, when the synthesized tag-fusion target proteins are arrayed on a chip, anti-tag antibodies for the fused tags can be commonly used regardless of the types of target proteins. This can provide an anti-tag antibody chip prepared in accordance with the relevant types of anti-tag antibodies as a product.

The present embodiment can be applied as a platform for interaction analysis of proteins on a solid phase, since interaction analysis of proteins can be conducted after the proteins are arrayed on a solid phase in a state where the structures and the functions thereof are maintained regardless of protein type. Thus, by combining peripheral devices necessary for analysis, such as a detection mechanism (Fig. 2) and a sample dispensing mechanism (Fig. 3), a full automatic or semiautomatic protein interaction analysis system can be provided.

Regarding the detection of interaction, in a case where a fluorescence-labeled sample is detected, a fluorescent scanner, a two-photon excitation scanner, or the like can be applied, for example. In contrast, in a case of a non-fluorescence-labeled sample, a surface plasmon resonance apparatus, an optical waveguide apparatus, or the like can be applied, for example.

Regarding the dispensing of anti-tag antibodies, tag-fusion target proteins, substances causing an interaction therewith, and the like, to a solid phase, a full automatic or semiautomatic dispensing processing system comprising a contact-type or non-contact-type sample dispensing mechanism, for example, can be constructed, in addition to manual procedures using a micro-pipetter, for example.

In a case where it is necessary to repeatedly dispense a sample at the same spot on a chip, a mark for positional recognition is set on the chip in advance, for example. The position of the spot is stored and determined through the recognition of the mark on the chip via a positioning apparatus (such as a CCD camera) above a sample dispensing apparatus.

Regarding operations such as blocking, washing, and the like concerning the surface of the chip, a full automatic or semiautomatic processing system comprising a hybridization apparatus, for example, can be constructed, in addition to manual procedures using a tray, for example.

The present embodiment allows a complicated protein interaction to be comprehensively and universally realized on a chip, utilizing genetic engineering methods that have spread and progressed in recent years. Thus, the present embodiment can be applied not merely to research use relating to proteins, but also to screening in medicine, examination, diagnosis, and the field of drug development. In particular, proteins are functional molecules that are actually in control of various actions in a living thing, unlike information molecules such as genes. Thus, the capability of realizing the interaction thereof on the chip comprehensively and universally is effective.

Also, the number of spots for the tag-fusion target proteins can be arbitrarily changed within the field of an arrayed surface of a solid phase used, so that densification and downsizing resulting therefrom become possible. Target proteins may be of any type, so that any protein can be immobilized on the chip in accordance with the principle of the present embodiment and can be used for interaction analysis. Thus, the present embodiment can conduct function analysis, such as screening, quantitation, expression profiling, or the like, with respect to the target proteins, and can also be applied to structure analysis.

The present embodiment is capable of arraying proteins on a solid phase with high density in a state where the structures and the functions thereof are maintained, and of allowing interaction analysis to be conducted. Thus, this technique may also be suitable for expression profiling of proteins. The state of functional molecules inside a living thing is more precisely reflected by directly examining the expression level of proteins resulting from translation than by examining the expression level of the transcription products of genes inside the living thing, as in a case involving a DNA microarray. Thus, interaction analysis and expression profiling via a protein chip are expected to spread in the future. The present embodiment can provide a platform therefor, and is suitable for contract analytical services for protein interaction analysis.

### (Verification Experiment)

### (Purpose of Experiment)

It is known that Ec DOS protein binds to PAS domain protein constituting a portion thereof through interaction (Yoshimura, T. et al., The Journal of Biological Chemistry, 278(52), 53105-53111 (2003)). Then, as shown in Fig. 5, after both thereof are prepared by a genetic engineering method, tag-fusion Ec DOS proteins are arrayed on a chip and the interaction of PAS domain proteins depending on concentration is examined.

### (Preparation of a tag-fusion Escherichia coli-derived direct oxygen sensor protein (Ec DOS) and a tag-fusion protein of heme-binding PAS domain constituting the Ec DOS)

An open reading frame for coding an Ec DOS protein is cloned by adjusting a frame to the multiple cloning site of a pET28a(+) expression vector (Novagen). In the same manner, a PAS domain constituting the Ec DOS protein is separately cloned. After each protein is independently over-expressed in a BL21 competent cell (Stratagene), the cell is lysed to recover a supernatant thereof. After ammonium sulfate precipitation, an eluate gained via a desalting process using a Sephadex G-25 column (Amersham Biosciences) is subjected to a Ni-NTA-agarose column (Qiagen), whereby each histidine-tag fusion protein is affinity purified. Concerning a tag-fusion PAS domain protein, a histidine-tag portion is separated by thrombin digestion, then a product thereof is subjected to a nickel chelating, thereby recovering a PAS domain protein as a supernatant in which a tag is removed. The gained PAS domain protein is fluorescence-labeled using a Cy3 labeling kit (Amersham Biosciences).

### (Preparation of an anti-tag antibody chip)

An anti-tag antibody chip is prepared in accordance with the procedure of Fig. 4. A commercially available protein chip (ProteoChip A, Hitachi High-Technologies) is used as a chip for immobilizing anti-tag antibodies. The ProteoChip A comprises a washed slide glass treated with an aminosilane coupling, followed by the coating with calixarene molecules of a protein immobilizing reagent. In the present embodiment, a spot seal (design registration applied for) in which holes of φ 2.0mm for spotting are provided in advance is attached to the chip, and a sample is dispensed in the spots, thereby conducting interaction analysis of target proteins. In the present embodiment, anti His-tag monoclonal antibody (MAB050, R&D Systems) are used as anti-tag antibody. The antibodies are prepared to have a concentration of 100 µg/ml using a dilution solution for antibody (PBS buffer prepared to have a pH of 7.4 including 30 % of glycerol). After dispensing 1.5 µl to each spot, the antibodies are immobilized on the chip through overnight incubation at 37 °C. The chip is steeped in a washing solution A (PBS buffer prepared to have a pH of 7.8 including 0.5 % of Tween-20) and excessive antibodies are removed while shaking it for 20 minutes. Then, the chip is rinsed with a deionized water. Next, the chip is steeped in a BSA solution of 3 % prepared by a PBS buffer (pH 7.4), and blocking is carried out while shaking it for 60 minutes at room temperature. The chip after the blocking is steeped in the washing solution A and washed while shaking it for 20 minutes. Then, the chip is rinsed with a deionized water, thereby preparing the anti-tag antibody chip.

### (Preparation of a chip for interaction analysis by the immobilization of a tag-fusion Ec DOS protein)

After dispensing 1.5 µl of tag-fusion Ec DOS proteins to each spot of the anti-tag antibody chip, the tag-fusion Ec DOS proteins being prepared to have a concentration of 100 µg/ml via a dilution solution for protein (Tris buffer prepared to have a pH of 7.4 including 10 % of BSA and 30 % of glycerol), the proteins are incubated for three hours at room temperature. The chip is steeped in a washing solution B (Tris buffer prepared to have a pH of 7.4 including 0.5 % of Tween-20) and excessive tag-fusion target proteins are removed while shaking it for 20 minutes. Then, the chip is rinsed with a deionized water, thereby preparing the chip for interaction analysis in which the tag-fusion Ec DOS proteins are arrayed.

### (Interaction analysis of the Ec DOS proteins and the PAS domain proteins)

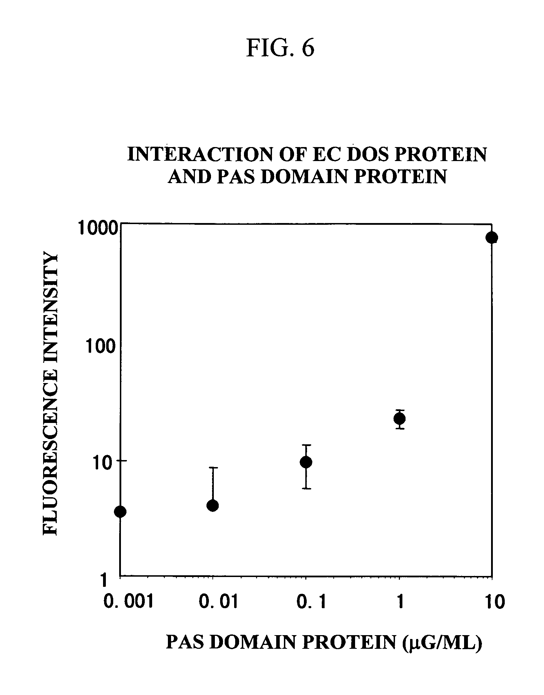

To the spots of the chip for interaction analysis in which the tag-fusion Ec DOS proteins are arrayed, 1.5 µl of Cy3-labeled PAS domain proteins is dispensed, the Cy3-labeled PAS domain proteins being diluted via a dilution solution for protein in five levels in a range of 0 to 10 µg/ml, and the proteins are incubated for three hours at room temperature. Then, the chip is steeped in the washing solution B and washed while shaking it for 20 minutes. Thereafter, the chip is rinsed with a deionized water, thereby removing those Cy3-labeled PAS domain proteins that are not interacted. Droplets on the chip are removed via the blowing of nitrogen gas, and then fluorescence detection is conducted via a fluorescence scanner (ScanArray Express, Perkin Elmer). Fig. 6 shows the results.

According to the results of Fig. 6, fluorescence intensity varies depending on the concentration of the Cy3-labeled PAS domain proteins. Thus, it is confirmed that the present embodiment realizes the interaction of the Ec DOS proteins and the PAS domain proteins on the chip. This suggests that target proteins can be arrayed on a chip in a state where the structures and the functions thereof are maintained, and that, by causing substances that interact therewith to react, the interaction of both of them can be monitored. With the use of high integration as one of the merits of using a chip, after a chip in which target proteins in drug development are arrayed with high density is prepared, for example, by collectively causing low-molecule compounds of many candidates for drug to react with each of the protein spots on the chip, separately, it becomes possible to screen those low-molecule compounds that show interaction.

The present invention can be applied not merely to research use relating to proteins, but also to medicine, examination, diagnosis, and the field of drug development.

## Claims

1. An anti-tag antibody chip comprising a layer including an anti-tag antibody formed on a substrate.

2. A method for manufacturing an anti-tag antibody chip, comprising washing and drying a substrate for an anti-tag antibody chip, processing the washed substrate with an aminosilane compound, processing the aminosilane substrate with a protein immobilizing reagent, and processing the substrate coated with the protein immobilizing reagent with an anti-tag antibody.

3. The method for manufacturing an anti-tag antibody chip according to claim 2, wherein the aminosilane compound comprises 3-aminopropyltriethoxysilane and the protein immobilizing reagent comprises calixarene.

4. The method for manufacturing an anti-tag antibody chip according to claim 2, comprising, after the substrate coated with the protein immobilizing reagent is processed with the anti-tag antibody, blocking a portion of the surface of the substrate where the anti-tag antibody is not immobilized using a blocking agent.

5. A tag-fusion target protein array comprising a tag-fusion target protein immobilized on a substrate via a layer including an anti-tag antibody.

6. The tag-fusion target protein array according to claim 5, wherein the tag-fusion target protein comprises a peptide or a polypeptide in the form of a tag added to an end of either an N-terminal or a C-terminal thereof or within the amino acid sequence of a target protein, depending on necessity, via a genetic engineering method.

7. A method for manufacturing a tag-fusion target protein array, comprising manufacturing an anti-tag antibody chip, and immobilizing a tag-fusion target protein on the anti-tag antibody chip via a layer including an anti-tag antibody.

8. The method for manufacturing a tag-fusion target protein array according to claim 7, wherein the tag-fusion target protein comprises a peptide or a polypeptide in the form of a tag added to an end of either an N-terminal or a C-terminal thereof or within the amino acid sequence of a target protein, depending on necessity, via a genetic engineering method.

9. A method for analyzing a protein interaction, comprising causing a solution including an interaction substance to react with a tag-fusion target protein array, wherein a tag-fusion target protein is arrayed on a substrate via a layer including an anti-tag antibody.

10. The method for analyzing a protein interaction according to claim 9, wherein the tag-fusion target protein comprises a peptide or a polypeptide in the form of a tag added to an end of either an N-terminal or a C-terminal thereof or within the amino acid sequence of a target protein, depending on necessity, via a genetic engineering method.

11. A platform for protein interaction analysis, comprising a tag-fusion target protein arrayed on an anti-tag antibody chip.

12. A method for purifying a tag-fusion target protein, comprising directly subjecting a product before purification expressed in a genetic engineering manner to an anti-tag antibody chip, and washing and removing debris in a state where only a tag-fusion target protein is bonded to an anti-tag antibody.

13. A protein interaction analysis apparatus comprising an anti-tag antibody chip mount mechanism for mounting an anti-tag antibody chip and moving thereof, a dispensing mechanism for dispensing a sample including a tag-fusion target protein to the anti-tag antibody chip, a position recognition mechanism capable of dispensing to the same spot on the chip in a repeated manner, and a reading mechanism of a labeled sample on the anti-tag antibody chip after reaction.

14. The protein interaction analysis apparatus according to claim 13, wherein the labeled sample comprises a fluorescence-labeled sample and the reading mechanism comprises a fluorescence scanner or a two-photon excitation scanner.

15. The protein interaction analysis apparatus according to claim 13, wherein the labeled sample comprises a non-fluorescence-labeled sample and the reading mechanism comprises a surface plasmon resonance (SPR) apparatus or an optical waveguide detection apparatus.
